# EUROPEAN PATENT SPECIFICATION

(11) **EP 3 731 816 B1**
(45) Date of publication and mention of the grant of the patent: **05.11.2025**
(21) Application number: 18899012.1
(22) Date of filing: 27.12.2018
(51) Int. Cl.: A61K 31/4545

(54) **SOLID ORAL PHARMACEUTICAL COMPOSITIONS COMPRISING APIXABAN**
FESTE ORALE PHARMAZEUTISCHE ZUSAMMENSETZUNGEN MIT APIXABAN
COMPOSITIONS PHARMACEUTIQUES ORALES SOLIDES COMPRENANT DE L'APIXABAN

(30) Priority: 28.12.2017 TR 201722523
(43) Date of publication of application: 04.11.2020
(73) Proprietor: Sanovel Ilac Sanayi ve Ticaret A.S., Sisli - Istanbul (TR)
(72) Inventor: TÜRKYILMAZ, Ali, 34460 Istanbul (TR); ZENGINER, Sibel, 34460 Istanbul (TR); YANC, Burcin, 34460 Istanbul (TR); ÖZTÜRK, Erkin, 34460 Istanbul (TR)
(74) Representative: Genç Ilhan, Oznur
(86) International application number: PCT/TR2018/050915
(87) International publication number: WO 2019/151965

(56) References cited:
- EP-A1- 3 243 505
- WO-A1-2017/088841
- CN-A- 105 943 536
- "Amorphous Formulations of Apixaban by Hot Melt Extrusion", RESEARCH DISCLOSURE, KENNETH MASON PUBLICATIONS, HAMPSHIRE, UK, GB, vol. 605, no. 34, 1 September 2014 (2014-09-01), pages 5, XP007143402, ISSN: 0374-4353
- URVE PAAVER ET AL: "Soluplus Graft Copolymer: Potential Novel Carrier Polymer in Electrospinning of Nanofibrous Drug Delivery Systems for Wound Therapy", BIOMED RESEARCH INTERNATIONAL, vol. 2014, 1 January 2014 (2014-01-01), pages 1 - 7, XP055622153, ISSN: 2314-6133, DOI: 10.1155/2014/789765

## Description

### Field of the Invention

The present invention relates to solid pharmaceutical compositions comprising apixaban or a pharmaceutically acceptable salt thereof and at least one pharmaceutically acceptable polymer. The composition is obtained by using an effective process.

### Background of the Invention

Apixaban is a highly selective, orally bioavailable, and reversible direct inhibitor of free and clot-bound factor Xa. Factor Xa catalyzes the conversion of prothrombin to thrombin, the final enzyme in the coagulation cascade that is responsible for fibrin clot formation. Apixaban has no direct effect on platelet aggregation, but by inhibiting factor Xa, it indirectly decreases clot formation induced by thrombin.

The chemical name of apixaban is 1-(4-methoxyphenyl)-7-oxo-6-[4-(2-oxopiperidin-1-yl)phenyl]-4,5,6,7-tetrahydro-1H-pyrazolo[3,4-c]pyridine-3-carboxamide and its chemical structure is shown in the Formula 1.

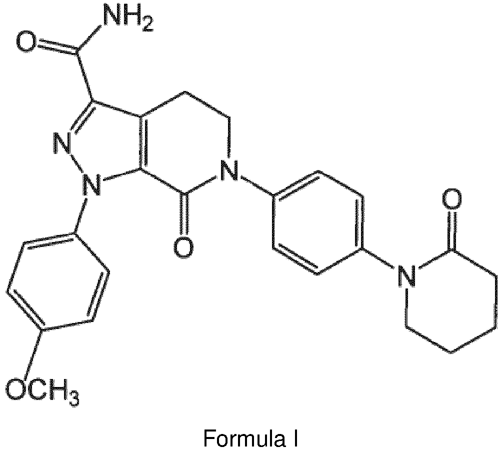

Apixaban, sold under the tradename Eliquis, is an anticoagulant for the treatment of venous thromboembolic events. It is taken orally. It is a direct factor Xa inhibitor and apixaban has poor solubility in water (0.028 mg/mL at 24 °C) and a relatively low oral bioavailability (about 50% for a single 10 mg dose).

Apixaban molecule is disclosed in U.S. Patent No. 6,967,208.

In prior art, there are patents which disclose oral pharmaceutical dosage forms comprising apixaban. WO2011106478 PCT application discloses a pharmaceutical composition comprises apixaban and a pharmaceutically acceptable diluent or carrier, wherein the apixaban is in particulate and crystalline form and the individual apixaban particles, whether the particles exist singly or are agglomerated, have a D90 equal to or less than 89 µm as measured by laser light scattering.

It was recognized that because of the targeted low dose for apixaban, content uniformity of the final product becomes an important product attribute. (European Medicine Agency, Assesment Report, Eliquis, 2011, Procedure No. EMEA/H/C/002148, page 10) Content uniformity is a pharmaceutical analysis parameter for the quality control of capsules and tablets. In this invention, the pharmaceutical compositions which have low dose of apixaban, have been developed with a desired uniformity of content.

U.S. patent US9,045,473 discloses apixaban in an amorphous form and processes for preparing an amorphous form of apixaban.

PCT application WO2013174498 discloses oral dosage forms for modified release of apixaban and methods of preparing said dosage forms.

"Amorphous Formulations of Apixaban by Hot Melt Extrusion", Research Disclosure, Kenneth Mason Publications, vol.605, no.34 (XP007143402) discloses apixaban formulations. XP007143402 describes apixaban formulation that comprises Soluplus polymer, copovidone, lactose anhydrous, microcrystalline cellulose, croscarmellose sodium, magnesium stearate and Opadry coating. According to the disclosure on page 1 in XP007143402; hot melt extrusion is one of the methods for preparing apixaban formulations wherein apixaban is in the amorphous form. On page 3 of XP007143402, the use of Soluplus ^{®} and copovidone at different ratios are mentioned.

There is still a need in the art to provide an improved oral pharmaceutical composition of apixaban, having a content uniformity, high solubility and therefore a high bioavailability and a long-term stability.

Therefore, there is an unmet need for new pharmaceutical composition comprising apixaban that exhibits improved dissolution rate, as a property optionally and preferably independent from a particle size that may be used.

### Detailed description of the Invention

The main object of the present invention is to provide pharmaceutical compositions have high solubility, high bioavailability, high physical and chemical stability and a long shelf life by using an effective process.

A further object of the present invention is to provide pharmaceutical compositions have high content uniformity.

The term "apixaban" as used herein refers to apixaban in the form of free base or in the form of pharmaceutically acceptable salts, crystalline polymorph, solvates, hydrates, esters or in an amorphous form or mixtures thereof.

Apixaban has poor solubility in water (0.028 mg/mL at 24 °C) and a relatively low oral bioavailability (about 50% for a single 10 mg dose).

At present invention, the pharmaceutical composition is suitable for oral administration. Solid oral dosage is used for bioavailability. Suitable solid oral dosage forms are selected from the group comprising tablets, capsules, granules, powders, pellet or unit dose packets.

In one embodiment, the solid oral dosage form is tablet.

In this invention, pharmaceutical compositions comprise apixaban and at least one pharmaceutically acceptable polymer, wherein the composition is obtained by means of a hot-melt method and wherein the composition further comprises microcrystalline cellulose is in an amount between 45.00% and 70.00% by weight of the total composition and dibasic anhydrous calcium phosphate as a diluent.

Hot-melt extrusion (HME) technology is prominent in the pharmaceutical industry. HME offers the potential of shorter and more efficient times to the final product, through reduction of the processing steps involved. HME is used to disperse active agent in a matrix at the molecular level, thus forming solid solutions. This method is used for poorly soluble active agent and obtained desired dissolution rate and stability.

Hot-melt extrusion is a technique for manufacturing amorphous solid dispersions in which the active agent is melted or dissolved within a dispersion carrier and mixed to produce and stabilize.

In one embodiment, the weight ratio of apixaban to the pharmaceutically acceptable polymer is between 0.1 and 0.5, preferably between 0.2 and 0.4.

The method described above both serves to provide a uniform formulation content, and eliminates the need for any liquid solvent including water. Any flowability-related problems encountered during production are prevented as well. In said formulation, the weight ratio of apixaban to pharmaceutically acceptable polymer ranges allow to achieve the desired dissolution rate and solubility. Polymers with low glass transition temperature and melting point are used in said formulation.

In one embodiment, the amount of apixaban is between 0.25% and 5.00%, preferably between 1.80% and 3.20% by weight of the total composition.

Suitable pharmaceutically acceptable polymers are selected from a group comprising graft copolymer of polyvinyl caprolactam, polyvinyl acetate, polyethylene glycol, homopolymers and copolymers of polyalkylene oxides, polyethylene glycol and polypropylene glycol, homopolymers and copolymers of N-vinyl lactams, N-vinylpyrrolidone, polyvinylpyrrolidone (PVP), N-vinylcaprolactam, homopolymers and copolymers of acrylic acid and its derivatives, homopolymers and copolymers of methacrylic acid and its derivatives, methylmethacrylate, methylcellulose, ethylcellulose, hydroxymethylcellulose, hydroxyethylcellulose (HEC), hydroxypropylcellulose (HPC), hydroxypropyl methylcellulose (HPMC), carboxymethylcellulose, starch derivatives or mixtures thereof.

In one embodiment, the pharmaceutically acceptable polymer is graft copolymer of polyvinyl caprolactam, polyvinyl acetate or polyethylene glycol or mixtures thereof (Soluplus).

In one embodiment, the composition further comprises at least one pharmaceutically acceptable excipient which is selected from diluents, disintegrants, lubricants, glidants, plasticizers or mixtures thereof.

The pharmaceutical composition of apixaban comprises dibasic anhydrous calcium phosphate as a diluent.

In another embodiment, the pharmaceutical composition of apixaban may comprise additional diluents selected from a group comprising microcrystalline cellulose, lactose, starch, mannitol, calcium phosphate dihydrate, calcium phosphate trihydrate, dibasic calcium phosphate, calcium carbonate, calcium sulfate, carboxymethyl cellulose calcium, powdered cellulose, cellulose acetate, pregelatinized starch, lactose monohydrate, corn starch or mixtures thereof.

In another embodiment, the pharmaceutical composition may comprise two different diluents.

According to another embodiment, the diluents may be dibasic anhydrous calcium phosphate and microcrystalline cellulose.

Choosing dibasic anhydrous calcium phosphate as diluent provides good flow and compaction properties and helps to ensure high content uniformity.

In one embodiment, the amount of dibasic anhydrous calcium phosphate is between 1.00% and 40.00%, preferably between 10.00% and 30.00%, more preferably between 15.00% and 25.00% by weight of the total composition.

In the present invention, the amount of microcrystalline cellulose is between n 45.00% and 70.00% by weight of the total composition.

Suitable disintegrants are selected from a group comprising cross-linked carboxymethyl cellulose sodium (croscarmellose sodium), pregelatinized starch, low-substituted hydroxypropyl cellulose (L-HPC), microcrystalline cellulose, sodium starch glycolate, starch, crospovidone (cross-linked polyvinil pyrrolidone), povidone, poloxomer, sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminum silica, sodium dodesyl sulphate, poloxamer, sodium glycine carbonate, sodium lauryl sulphate or mixtures thereof.

In one preferred embodiment, the disintegrant is cross-linked carboxymethyl cellulose sodium (croscarmellose sodium).

According to these embodiments, the amount of disintegrant is between 1.00% and 10.00%, preferably between 2.00% and 7.50%, more preferably between 2.00% and 5.00% by weight of the total composition.

Suitable lubricants are selected from a group comprising magnesium stearate, sodium stearyl fumarate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium lauryl sulphate or mixtures thereof.

In one preferred embodiment, the lubricant is magnesium stearate.

According to these embodiments, the amount of lubricant is between 0.10% and 5.00%, preferably between 0.25% and 2.50%, more preferably between 0.50% and 1.50% by weight of the total composition.

Suitable glidants are selected from a group comprising colloidal silicon dioxide, talc, aluminum silicate or mixtures thereof.

In one preferred embodiment, the glidant is colloidal silicon dioxide.

According to these embodiments, the amount of glidant is between 0.10% and 5.00%, preferably 0.25% and 2.50%, more preferably between 0.50% and 1.50% by weight of the total composition.

Suitable coating materials are selected from the group comprising hydroxypropylmethyl cellulose (hypromellose), hydroxypropyl cellulose, polyvinyl alcohol (PVA), polyethylene glycol (PEG), talc, polyvinyl alcohol-polyethylene glycol copolymers (Kollicoat IR), polyvinylprolidone, polyvinylprolidone-vinyl acetate copolymer (PVP-VA) and all kinds of OpadryTM, pigments, dyes, titanium dioxide, iron oxide or polymethylmetacrylate copolymers (Eudragit) or mixtures thereof.

In one preferred embodiment, the plasticizer is selected from polyoxyethylene sorbitan fatty acid esters which are polysorbate 20, polysorbate 21, polysorbate 40, polysorbate 60, polysorbate 61, polysorbate 65, polysorbate 80, polysorbate 81, polysorbate 85, polysorbate 120 or mixtures thereof.

In one preferred embodiment, the plasticizer is polysorbate 80 (Tween 80).

In one embodiment, during the hot melt extrusion method, the temperature is decreased by using plasticizer.

Using a plasticizer reduces the glass transition temperature and it increases the stability of the active agent. The plasticizer used in a hot-melt method drops down the glass transition temperature of the polymers used in hot-melting, and thus allows to formulate the active agent at lower temperatures. In result, the formulation is made more stable.

Preferably heating is limited to provide a temperature no greater than the fluxing temperature of the admixture to ensure homogeneity of the composition before cooling the melt to provide a solid.

The temperature is chosen according to the properties of the polymers and apixaban.

**Table 1: Temperatures zones along hot melt extrusion**

| Zone 1 | Zone 2 | Zone 3 | Zone 4 | Zone 5 | Zone 6 | Zone 7 | Zone 8 |
|---|---|---|---|---|---|---|---|
| 20-80 °C | 60-120 °C | 100-180 °C | 120-200 °C | 140-220 °C | 150-230 °C | 155-235 °C | 160-240 °C |

The temperature ranges given above is suitable at this invention to provide high solubility, high physical and chemical stability.

In one preferred embodiment, the pharmaceutical composition comprises apixaban as active agent, graft copolymer of polyvinyl caprolactam, polyvinyl acetate, polyethylene glycol as pharmaceutically acceptable polymer, dibasic anhydrous calcium phosphate and microcrystalline cellulose as diluent, cross-linked carboxymethyl cellulose sodium (croscarmellose sodium) as disintegrant, colloidal silicon dioxide as glidant, magnesium stearate as lubricant and a coating layer.

According to this preferred embodiment, the composition comprises;
a. 0.25-5.00% by weight apixaban
b. 0.75-15.00% by weight graft copolymer of polyvinyl caprolactam, polyvinyl acetate, polyethylene glycol
c. 1.00-10.00% by weight croscarmellose sodium
d. 1.00-40.00% by weight dibasic anhydrous calcium phosphate
e. 45.00-70.00% by weight microcrystalline cellulose
f. 0.10-5.00% by weight colloidal silicon dioxide
g. 0.10-5.00% by weight magnesium stearate
h. 1.00-5.00% by weight coating

According to this preferred embodiment, the composition comprises;
a. 1.80-3.20% by weight apixaban
b. 5.50-9.50% by weight graft copolymer of polyvinyl caprolactam, polyvinyl acetate, polyethylene glycol
c. 1.00-5.00 polysorbate 80
d. 1.00-5.00% by weight croscarmellose sodium
e. 15.00-25.00% by weight dibasic anhydrous calcium phosphate
f. 45.00-70.00% by weight microcrystalline cellulose
g. 0.10-5.00% by weight colloidal silicon dioxide
h. 0.10-5.00% magnesium stearate
i. 1.00-5.00% by weight coating

Feed rate and screw rate values are important for providing homogeneity in the extrudate.

However, screw rate value provides minimizing the risk for thermal degradation of apixaban. The following values give the best stability result for the composition.

The temperature of the hot melt extrusion is set in the range of about 40° C to about 250° C and the rotation speed of the screw is set in the range of about 100 to about 350 rpm.

At this invention, the rotation speed of the screw is between 100 rpm and 150 rpm, 150 rpm and 200 rpm, 200 rpm and 250 rpm, 250 rpm and 300 rpm, 300 rpm and 350 rpm.

At this invention, the feed rate during hot melt extrusion process is between 10 g/min and 15 g/min, 15 g/min and 20 g/min, 20 g/min and 25 g/min, 25 g/min and 30 g/min, 30 g/min and 35 g/min.

At this invention, the torque value during hot melt extrusion process is between 20% and 30%.

The compositions of invention can be developed into oral dosage form comprising immediate release, extended release, sustained release, controlled release, modified release and delayed release or combination thereof. Such compositions can be prepared by using rate controlling polymers.

### Example 1: Tablet

| **Ingredients** | | **(%) amount (w/w)** |
|---|---|---|
| Extrudate | Apixaban 25.00% | |
| | Soluplus 75.00% | 7.50-12.50 |
| Croscarmellose sodium | | 1.00-5.00 |
| Dibasic anhydrous calcium phosphate | | 15.00-25.00 |
| Microcrystalline cellulose | | 45.00-70.00 |
| Colloidal silicon dioxide | | 0.10-5.00 |
| Magnesium stearate | | 0.10-5.00 |
| **Total** | | **100** |
| Coating | | 1.00-5.00 |
| **Total coated tablet** | | 101.00-105.00 |

| | | |
|---|---|---|
| Soluplus: graft copolymer of polyvinyl caprolactam, polyvinyl acetate, polyethylene glycol Tween 80: Polysorbate 80 | | |

### Example 2: Tablet

| **Ingredients** | **(%) amount (w/w)** |
|---|---|
| Apixaban | 1.80-3.20 |
| Soluplus | 5.50-9.50 |
| Croscarmellose sodium | 1.00-5.00 |
| Dibasic anhydrous calcium phosphate | 15.00-25.00 |
| Microcrystalline cellulose | 45.00-70.00 |
| Colloidal silicon dioxide | 0.10-5.00 |
| Magnesium stearate | 0.10-5.00 |
| **Total** | **100** |
| Coating | 1.00-5.00 |
| **Total coated tablet** | 101.00-105.00 |

### Example 3: Tablet

| **Ingredients** | | **(%) amount (w/w)** |
|---|---|---|
| Extrudate | Apixaban 25.00% | |
| | Soluplus 75.00% | 1.00-20.00 |
| Croscarmellose sodium | | 1.00-10.00 |
| Dibasic anhydrous calcium phosphate | | 1.00-40.00 |
| Microcrystalline cellulose | | 45.00-70.00 |
| Colloidal silicon dioxide | | 0.10-5.00 |
| Magnesium stearate | | 0.10-5.00 |
| **Total** | | **100** |
| Coating | | 1.00-5.00 |
| **Total coated tablet** | | 101.00-105.00 |

### Example 4: Tablet

| **Ingredients** | **(%) amount (w/w)** |
|---|---|
| Apixaban | 0.25-5.00 |
| Soluplus | 0.75-15.00 |
| Croscarmellose sodium | 1.00-10.00 |
| Dibasic anhydrous calcium phosphate | 1.00-40.00 |
| Microcrystalline cellulose | 45.00-70.00 |
| Colloidal silicon dioxide | 0.10-5.00 |
| Magnesium stearate | 0.10-5.00 |
| **Total** | **100** |
| Coating | 1.00-5.00 |
| **Total coated tablet** | 101.00-105.00 |

The pharmaceutical formulations subjected to the invention are prepared in detail by following these steps:
Preparation of extrudate
   a. Mixing apixaban and Soluplus
   b. Melting this mixture in a Hot melt extruder for molecular dispersion of apixaban in the polymer to form extrudate
Preparation of tablets
   a. Pulverising and sieving the extrudate
   b. Mixing the extrudate powder, croscarmellose sodium, anhydrous dibasic calcium phosphate and microcrystalline cellulose
   c. Sieving colloidal silicon dioxide and adding to the mixture
   d. Adding magnesium stearate to the mixture and mixing
   e. Compressing the mixture into tablets
   f. Coating these tablets

### Example 5: Tablet

| **Ingredients** | | **(%) amount (w/w)** |
|---|---|---|
| Extrudate | Apixaban 25.0% | |
| | Soluplus 73.0% | |
| | Tween 80 2.0% | 7.50-12.50 |
| Croscarmellose sodium | | 1.00-5.00 |
| Dibasic anhydrous calcium phosphate | | 15.00-25.00 |
| Microcrystalline cellulose | | 45.00-70.00 |
| Colloidal silicon dioxide | | 0.10-5.00 |
| Magnesium stearate | | 0.10-5.00 |
| **Total** | | **100** |
| Coating | | 1.00-5.00 |
| **Total coated tablet** | | 101.00-105.00 |

### Example 6: Tablet

| **Ingredients** | **(%) amount (w/w)** |
|---|---|
| Apixaban | 1.80-3.20 |
| Soluplus | 5.50-9.50 |
| Tween 80 | 1.00-5.00 |
| Croscarmellose sodium | 1.00-5.00 |
| Dibasic anhydrous calcium phosphate | 15.00-25.00 |
| Microcrystalline cellulose | 45.00-70.00 |
| Colloidal silicon dioxide | 0.10-5.00 |
| Magnesium stearate | 0.10-5.00 |
| **Total** | **100** |
| Coating | 1.00-5.00 |
| **Total coated tablet** | 101.00-105.00 |

The pharmaceutical formulations subjected to the invention are prepared in detail by following these steps:
Preparation of extrudate
   a. Mixing apixaban, Soluplus and Tween 80
   b. Melting this mixture in a Hot melt extruder for molecular dispersion of apixaban in the polymer to form extrudate
Preparation of tablets
   a. Pulverising and sieving the extrudate
   b. Mixing the extrudate powder, croscarmellose sodium, dibasic anhydrous calcium phosphate and microcrystalline cellulose
   c. Sieving colloidal silicon dioxide and adding to the mixture
   d. Adding magnesium stearate to the mixture and mixing
   e. Compressing the mixture into tablets
   f. Coating these tablets

## Claims

1. A pharmaceutical composition comprising apixaban and at least one pharmaceutically acceptable polymer, wherein the composition is obtained by means of a hot-melt method and wherein the composition further comprises microcrystalline cellulose is in an amount between 45.00% and 70.00% by weight of the total composition and dibasic anhydrous calcium phosphate as a diluent.

2. The pharmaceutical composition according to claim 1, wherein the weight ratio of apixaban to the pharmaceutically acceptable polymer is between 0.1 and 0.5, preferably between 0.2 and 0.4.

3. The pharmaceutical composition according to claim 1, wherein the amount of apixaban is between 0.25% and 5.00%, preferably between 1.80% and 3.20% by weight of the total composition.

4. The pharmaceutical composition according to claim 1, wherein said at least one pharmaceutically acceptable polymer is selected from a group comprising graft copolymer of polyvinyl caprolactam, polyvinyl acetate, polyethylene glycol, homopolymers and copolymers of polyalkylene oxides, polyethylene glycol and polypropylene glycol, homopolymers and copolymers of N-vinyl lactams, N-vinylpyrrolidone, polyvinylpyrrolidone , N-vinylcaprolactam, homopolymers and copolymers of acrylic acid and its derivatives, homopolymers and copolymers of methacrylic acid and its derivatives, methylmethacrylate, methylcellulose, ethylcellulose, hydroxymethylcellulose, hydroxyethylcellulose, hydroxypropylcellulose, hydroxypropyl methylcellulose, carboxymethylcellulose, starch derivatives or mixtures thereof.

5. The pharmaceutical composition according to claim 4, wherein said at least one pharmaceutically acceptable polymer is graft copolymer of polyvinyl caprolactam, polyvinyl acetate, polyethylene glycol.

6. The pharmaceutical composition according to claim 3, wherein the composition further comprises at least one pharmaceutically acceptable excipient which is selected from diluents, disintegrants, lubricants, glidants, plasticizers or mixtures thereof.

7. The pharmaceutical composition according to claim 1, wherein the amount of dibasic anhydrous calcium phosphate is between 1.00% and 40.00%, preferably between 10.00% and 30.00%, more preferably between 15.00% and 25.00% by weight of the total composition

8. The pharmaceutical composition according to claim 6, wherein the glidant is selected from a group comprising colloidal silicon dioxide, talc, aluminum silicate or mixtures thereof.

9. The pharmaceutical composition according to claim 8, wherein the glidant is colloidal silicon dioxide.

10. The pharmaceutical composition according to any of the preceding claims, wherein the composition comprising;
a. 0.25-5.00% by weight apixaban
b. 0.75-15.00% by weight graft copolymer of polyvinyl caprolactam, polyvinyl acetate, polyethylene glycol
c. 1.00-10.00% by weight croscarmellose sodium
d. 1.00-40.00% by weight dibasic anhydrous calcium phosphate
e. 45.00-70.00% by weight microcrystalline cellulose
f. 0.10-5.00% by weight colloidal silicon dioxide
g. 0.10-5.00% magnesium stearate
h. 1.00-5.00% by weight coating

11. A hot melt extrusion process for preparing the pharmaceutical composition according to claim 10, comprising the following steps:
a. Mixing apixaban and graft copolymer of polyvinyl caprolactam, polyvinyl acetate, polyethylene glycol
b. Melting this mixture in a hot melt extruder for molecular dispersion of apixaban in the polymer to form extrudate
c. Pulverising and sieving the extrudate
d. Mixing the extrudate powder, croscarmellose sodium, dibasic anhydrous calcium phosphate and microcrystalline cellulose
e. Sieving colloidal silicon dioxide and adding to the mixture
f. Adding magnesium stearate to the mixture and mixing
g. Compressing the mixture into tablets
h. Coating these tablets

12. The pharmaceutical composition according to any one of the claims 1-10, wherein the composition comprising;
a. 1.80-3.20% by weight apixaban
b. 5.50-9.50% by weight graft copolymer of polyvinyl caprolactam, polyvinyl acetate, polyethylene glycol
c. 1.00-5.00 polysorbate 80
d. 1.00-5.00% by weight croscarmellose sodium
e. 15.00-25.00% by weight dibasic anhydrous calcium phosphate
f. 45.00-70.00% by weight microcrystalline cellulose
g. 0.10-5.00% by weight colloidal silicon dioxide
h. 0.10-5.00% magnesium stearate
i. 1.00-5.00% by weight coating

13. A hot melt extrusion process for preparing the pharmaceutical composition according to claim 12, comprising the following steps:
a. Mixing apixaban, polysorbate 80 and graft copolymer of polyvinyl caprolactam, polyvinyl acetate, polyethylene glycol
b. Melting this mixture in a hot melt extruder for molecular dispersion of apixaban in the polymer to form extrudate
c. Pulverising and sieving the extrudate
d. Mixing the extrudate powder, croscarmellose sodium, dibasic anhydrous calcium phosphate and microcrystalline cellulose
e. Sieving colloidal silicon dioxide and adding to the mixture
f. Adding magnesium stearate to the mixture and mixing
g. Compressing the mixture into tablets
h. Coating these tablets

## Patentansprüche

1. Pharmazeutische Zusammensetzung, umfassend Apixaban und mindestens ein pharmazeutisch akzeptables Polymer, wobei die Zusammensetzung mittels eines Heißschmelzverfahrens erhalten wird und wobei die Zusammensetzung ferner mikrokristalline Cellulose in einer Menge zwischen 45,00 Gew.-% und 70,00 Gew.-% der Gesamtzusammensetzung und dibasisches wasserfreies Calciumphosphat als Verdünnungsmittel umfasst.

2. Pharmazeutische Zusammensetzung nach Anspruch 1, wobei das Gewichtsverhältnis von Apixaban zu dem pharmazeutisch akzeptablen Polymer zwischen 0,1 und 0,5, vorzugsweise zwischen 0,2 und 0,4 liegt.

3. Die pharmazeutische Zusammensetzung nach Anspruch 1, wobei die Menge an Apixaban zwischen 0,25 % und 5,00 %, vorzugsweise zwischen 1,80 % und 3,20 Gew.-% der Gesamtzusammensetzung beträgt.

4. Die pharmazeutische Zusammensetzung nach Anspruch 1, wobei das mindestens eine pharmazeutisch akzeptable Polymer aus einer Gruppe ausgewählt ist, die ein Pfropfcopolymer aus Polyvinylcaprolactam, Polyvinylacetat, Polyethylenglykol, Homopolymeren und Copolymeren von Polyalkylenoxiden, Polyethylenglykol und Polypropylenglykol, Homopolymeren und Copolymeren von N-Vinyllactamen, N-Vinylpyrrolidon, Polyvinylpyrrolidon, N-Vinylcaprolactam, Homopolymeren und Copolymeren von Acrylsäure und deren Derivaten, Homopolymeren und Copolymeren von Methacrylsäure und deren Derivaten, Methylmethacrylat, Methylcellulose, Ethylcellulose, Hydroxymethylcellulose, Hydroxyethylcellulose, Hydroxypropylcellulose, Hydroxypropylmethylcellulose, Carboxymethylcellulose, Stärkederivaten oder Mischungen davon enthalten.

5. Die pharmazeutische Zusammensetzung nach Anspruch 4, wobei das mindestens eine pharmazeutisch akzeptable Polymer ein Pfropfcopolymer aus Polyvinylcaprolactam, Polyvinylacetat und Polyethylenglykol ist.

6. Die pharmazeutische Zusammensetzung nach Anspruch 3, wobei die Zusammensetzung ferner mindestens einen pharmazeutisch akzeptablen Hilfsstoff umfasst, der aus Verdünnungsmitteln, Sprengmitteln, Schmiermitteln, Gleitmitteln, Weichmachern oder Mischungen davon ausgewählt ist.

7. Pharmazeutische Zusammensetzung nach Anspruch 1, wobei die Menge an dibasischem wasserfreiem Calciumphosphat zwischen 1,00 % und 40,00 %, vorzugsweise zwischen 10,00 % und 30,00 %, besonders bevorzugt zwischen 15,00 % und 25,00 %, bezogen auf das Gesamtgewicht der Zusammensetzung, beträgt.

8. Die pharmazeutische Zusammensetzung nach Anspruch 6, wobei das Gleitmittel aus einer Gruppe ausgewählt ist, die kolloidales Siliciumdioxid, Talk, Aluminiumsilikat oder Mischungen davon umfasst.

9. Die pharmazeutische Zusammensetzung nach Anspruch 8, wobei das Gleitmittel kolloidales Siliciumdioxid ist.

10. Die pharmazeutische Zusammensetzung nach einem der vorstehenden Ansprüche, wobei die Zusammensetzung umfasst:
a. 0,25 bis 5,00 Gew.-% Apixaban
b. 0,75 bis 15,00 Gew.-% eines Pfropfcopolymers aus Polyvinylcaprolactam, Polyvinylacetat und Polyethylenglykol
c. 1,00 bis 10,00 Gew.-% Croscarmellose-Natrium
d. 1,00 bis 40,00 Gew.-% dibasisches wasserfreies Calciumphosphat
e. 45,00-70,00 Gew.-% mikrokristalline Cellulose
f. 0,10-5,00 Gew.-% kolloidales Siliciumdioxid
g. 0,10-5,00 % Magnesiumstearat
h. 1,00-5,00 Gew.-% Überzug

11. Heißschmelzextrusionsverfahren zur Herstellung der pharmazeutischen Zusammensetzung nach Anspruch 10, umfassend die folgenden Schritte:
a. Mischen von Apixaban und einem Pfropfcopolymer aus Polyvinylcaprolactam, Polyvinylacetat und Polyethylenglykol
b. Schmelzen dieser Mischung in einem Heißschmelzextruder zur molekularen Dispersion von Apixaban im Polymer unter Bildung eines Extrudats
c. Pulverisieren und Sieben des Extrudats
d. Mischen des Extrudatpulvers, Croscarmellose-Natrium, dibasischem wasserfreiem Calciumphosphat und mikrokristalliner Cellulose
e. Sieben von kolloidalem Siliciumdioxid und Hinzufügen zur Mischung
f. Magnesiumstearat zu der Mischung hinzufügen und mischen
g. Die Mischung zu Tabletten pressen
h. Beschichten dieser Tabletten

12. Die pharmazeutische Zusammensetzung nach einem der Ansprüche 1 bis 10, wobei die Zusammensetzung umfasst:
a. 1,80-3,20 Gew.-% Apixaban
b. 5,50-9,50 Gew.-% Pfropfcopolymer aus Polyvinylcaprolactam, Polyvinylacetat, Polyethylenglykol
c. 1,00-5,00 % Gew.-Polysorbat 80
d. 1,00-5,00 Gew.-% Croscarmellose-Natrium
e. 15,00-25,00 Gew.-% dibasisches wasserfreies Calciumphosphat
f. 45,00-70,00 Gew.-% mikrokristalline Cellulose
g. 0,10-5,00 Gew.-% kolloidales Siliciumdioxid
h. 0,10-5,00 Gew.-% Magnesiumstearat
i. 1,00-5,00 Gew.-% Überzug

13. Heißschmelzextrusionsverfahren zur Herstellung der pharmazeutischen Zusammensetzung nach Anspruch 12, umfassend die folgenden Schritte:
a. Mischen von Apixaban, Polysorbat 80 und einem Pfropfcopolymer aus Polyvinylcaprolactam, Polyvinylacetat und Polyethylenglykol
b. Schmelzen dieser Mischung in einem Heißschmelzextruder zur molekularen Dispersion von Apixaban im Polymer, unter Bildung eines Extrudats
c. Pulverisieren und Sieben des Extrudats
d. Mischen des Extrudatpulvers, Croscarmellose-Natrium, dibasischem wasserfreiem Calciumphosphat und mikrokristalliner Cellulose
e. Sieben von kolloidalem Siliciumdioxid und Hinzufügen zur Mischung
f. Magnesiumstearat zu der Mischung hinzufügen und mischen
g. Die Mischung zu Tabletten pressen
h. Beschichten dieser Tabletten

## Revendications

1. - Composition pharmaceutique comprenant de l'apixaban et au moins un polymère pharmaceutiquement acceptable, la composition étant obtenue par une méthode de fusion à chaud et la composition comprenant en outre de la cellulose microcristalline dans une quantité entre 45,00 % et 70,00 % en poids de la composition totale et du phosphate de calcium anhydre dibasique en tant que diluant.

2. - Composition pharmaceutique selon la revendication 1, dans laquelle le rapport en poids de l'apixaban au polymère pharmaceutiquement acceptable est entre 0,1 et 0,5, de préférence entre 0,2 et 0,4.

3. - Composition pharmaceutique selon la revendication 1, dans laquelle la quantité d'apixaban est entre 0,25% et 5,00%, de préférence entre 1,80% et 3,20%, en poids de la composition totale.

4. - Composition pharmaceutique selon la revendication 1, dans laquelle ledit au moins un polymère pharmaceutiquement acceptable est choisi dans un groupe comprenant un copolymère greffé de polyvinyl caprolactame, de poly(acétate de vinyle), de polyéthylène glycol, les homopolymères et copolymères de poly(oxydes d'alkylène), de polyéthylène glycol et de polypropylène glycol, les homopolymères et copolymères de N-vinyl lactames, de N-vinylpyrrolidone, de polyvinylpyrrolidone, de N-vinylcaprolactame, les homopolymères et copolymères de l'acide acrylique et de ses dérivés, les homopolymères et copolymères de l'acide méthacrylique et de ses dérivés, le méthacrylate de méthyle, la méthylcellulose, l'éthylcellulose, l'hydroxyméthylcellulose, l'hydroxyéthylcellulose, l'hydroxypropylcellulose, l'hydroxypropyl méthylcellulose, la carboxyméthylcellulose, les dérivés de l'amidon ou leurs mélanges.

5. - Composition pharmaceutique selon la revendication 4, dans laquelle ledit au moins un polymère pharmaceutiquement acceptable est un copolymère greffé de polyvinyl caprolactame, de poly(acétate de vinyle), de polyéthylène glycol.

6. . Composition pharmaceutique selon la revendication 3, dans laquelle la composition comprend en outre au moins un excipient pharmaceutiquement acceptable qui est choisi parmi les diluants, les désintégrants, les lubrifiants, les agents de glissement, les plastifiants ou leurs mélanges.

7. - Composition pharmaceutique selon la revendication 1, dans laquelle la quantité de phosphate de calcium anhydre dibasique est entre 1,00 % et 40,00 %, de préférence entre 10,00 % et 30,00 %, de façon davantage préférée entre 15,00 % et 25,00 %, en poids de la composition totale.

8. - Composition pharmaceutique selon la revendication 6, dans laquelle l'agent de glissement est choisi dans un groupe comprenant le dioxyde de silicium colloïdal, le talc, le silicate d'aluminium ou leurs mélanges.

9. - Composition pharmaceutique selon la revendication 10, dans laquelle l'agent de glissement est le dioxyde de silicium colloïdal.

10. - Composition pharmaceutique selon l'une quelconque des revendications précédentes, dans laquelle la composition comprend :
a. 0,25-5,00% en poids d'apixaban
b. 0,75-15,00% en poids de copolymère greffé de polyvinyl caprolactame, de poly(acétate de vinyle), de polyéthylène glycol
c. 1,00-10,00% en poids de croscarmellose sodique
d. 1,00-40,00% en poids de phosphate de calcium anhydre dibasique
e. 45,00-70,00 % en poids cellulose microcristalline
f. 0,10-5,00 % en poids de dioxyde de silicium colloïdal
g. 0,10-5,00 % en poids stéarate de magnésium
h.1 .00-5.00% en poids d'enrobage.

11. - Procédé d'extrusion à chaud pour préparer la composition pharmaceutique selon la revendication 10, comprenant les étapes suivantes :
a. Mélange de l'apixaban et du copolymère greffé de polyvinyl caprolactame, de poly(acétate de vinyle), de polyéthylène glycol
b. Fusion de ce mélange dans une extrudeuse à chaud pour une dispersion moléculaire d'apixaban dans le polymère afin de former un extrudat
c. Pulvérisation et tamisage de l'extrudat
d. Mélange de la poudre de l'extrudat, de la croscarmellose sodique, du phosphate de calcium anhydre dibasique et de la cellulose microcristalline
e. Tamisage du dioxyde de silicium colloïdal et addition au mélange
f. Addition du stéarate de magnésium au mélange et mélange
g. Compression du mélange en comprimés
h. Enrobage de ces comprimés.

12. - Composition pharmaceutique selon l'une quelconque des revendications 1 à 10, dans laquelle la composition comprend :
a.1,80-3,20 % en poids d'apixaban
b. 5,50-9,50 % en poids de copolymère greffé de polyvinyl caprolactame, de poly(acétate de vinyle), de polyéthylène glycol
c. 1,00-5,00 % en poids de polysorbate 80
d. 1,00-5,00% en poids de croscarmellose sodique
e. 15,00-25,00% en poids de phosphate de calcium anhydre dibasique
f. 45,00-70,00 % en poids de cellulose microcristalline
g. 0,10-5,00 % en poids de dioxyde de silicium colloïdal
h. 0,10-5,00 % en poids de stéarate de magnésium
i. 1,00-5,00 % en poids d'enrobage.

13. - Procédé d'extrusion à chaud pour préparer la composition pharmaceutique selon la revendication 12, comprenant les étapes suivantes :
a. Mélange de l'apixaban, du polysorbate 80 et du copolymère greffé de polyvinyl caprolactame, de poly(acétate de vinyle), de polyéthylène glycol
b. Fusion de ce mélange dans une extrudeuse à chaud pour une dispersion moléculaire d'apixaban dans le polymère afin de former un extrudat
c. Pulvérisation et tamisage de l'extrudat
d. Mélange de la poudre de l'extrudat, de la croscarmellose sodique, du phosphate de calcium anhydre dibasique et de la cellulose microcristalline.
e. Tamisage du dioxyde de silicium colloïdal et addition au mélange
f. Addition du stéarate de magnésium au mélange et mélange
g. Compression du mélange en comprimés
h. Enrobage de ces comprimés.
